(19) Europäisches Patentamt
European Patent Office
Office européen des brevets

(11) Publication number: **0 306 264 A2**

(12) # EUROPEAN PATENT APPLICATION

(21) Application number: **88308011.1**

(22) Date of filing: **30.08.88**

(51) Int. Cl.[4]: **C 07 D 309/30**
C 07 C 69/757,
A 61 K 31/365, C 07 C 69/30,
A 61 K 31/22

(30) Priority: **02.09.87 US 92354**

(43) Date of publication of application:
**08.03.89 Bulletin 89/10**

(84) Designated Contracting States:
**AT BE CH DE ES FR GB GR IT LI LU NL SE**

(71) Applicant: **MERCK & CO. INC.**
**126, East Lincoln Avenue P.O. Box 2000**
**Rahway New Jersey 07065-0900 (US)**

(72) Inventor: **Hartman, George D.**
**1529 Tennis Circle**
**Lansdale§Pennsylvania 19446 (US)**

**Smith, Robert L.**
**1355 Pickwick Lane**
**Lansdale§Pennsylvania 19446 (US)**

(74) Representative: **Hesketh, Alan, Dr.**
**European Patent Department Merck & Co., Inc. Terlings Park Eastwick Road**
**Harlow Essex, CM20 2QR (GB)**

Claims for the following Contracting States: ES + GR.

(54) **Novel HMG-COA reductase inhibitors.**

(57) Novel 3-hydroxy-3-methylglutaryl-coenzyme A (HMG-CoA) reductase inhibitors are useful as antihypercholesterolemic agents and are represented by the following general structural formulae (I) and (II):

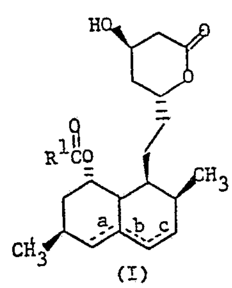

(I)

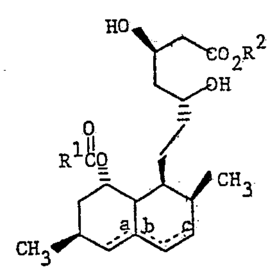

(II)

EP 0 306 264 A2

**Description**

## NOVEL HMG-CoA REDUCTASE INHIBITORS

### BACKGROUND OF THE INVENTION

Hypercholesterolemia is known to be one of the prime risk factors for atherosclerosis and coronary heart disease, the leading cause of death and disability in western countries. To date, there is still no effective antihypercholesterolemic agent commercially available that has found wide patient acceptance. The bile acid sequestrants seem to be moderately effective but they must be consumed in large quantities, i.e., several grams at a time, and they are not very palatable.

There are agents known, however, that are very active antihypercholesterolemic agents which function by limiting cholesterol biosynthesis via inhibiting the enzyme. HMG-CoA reductase. These agents include the natural fermentation products, such as mevastatin, lovastatin and provastatin and semisynthetic analogs, such as simvastatin. These compounds have the following chemical structural formulae:

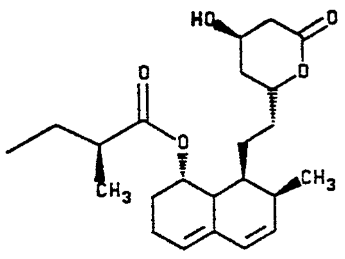

Mevastatin

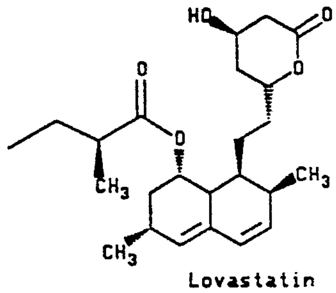

Lovastatin

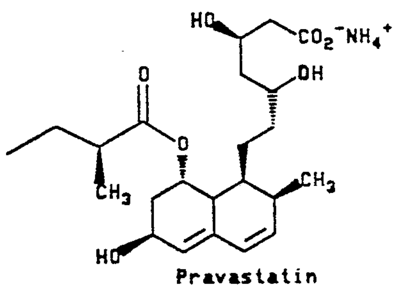

Pravastatin

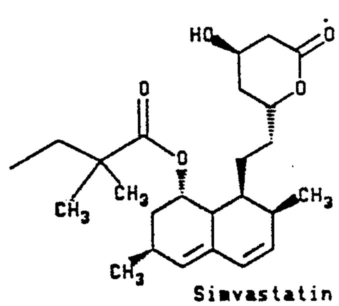

Simvastatin

Numerous analogs and homologs of these compounds have been described in the patent literature. U.S. Patent 4,444,784 discloses analogs of lovastatin which possess polyhydronaphthyl moieties and various 8-acyloxy groups attached thereto. U.S. Patent 4,661,483 also discloses analogs of lovastatin wherein the 8-acyloxy group has been elaborated. Additionally, co-pending U.S. applications Serial Numbers 859,513, 859,524, 859,525, 859,530, 859,534, and 859,535 all filed on May 5, 1986, disclose further analogs of lovastatin which have functionalized 8-acyloxy groups. All of the lovastatin analogs, including simvastatin, which contain a 6-methyl group have that substituent in the natural 6α or axial configuration.

Co-pending U.S. patent application, Serial No. 048,136 filed May 15, 1987, discloses compounds which are analogs of lovastatin and related compounds which possess a hydroxymethyl group, acyloxymethyl group, carbamoyloxymethyl group, a carboxy group, an alkoxycarbonyl group or a carbamoyl group substituted on the 6-position of the polyhydronaphthyl moiety. The compounds in this application may possess a substituent in the 6-position in either the 6α or 6β stereochemical position.

### SUMMARY OF THE INVENTION

This invention relates to novel compounds which are HMG-CoA reductase inhibitors and are useful as antihypercholesterolemic agents. Specifically, the compounds of this invention are analogs of lovastatin and related compounds which possess a methyl group in the 6-position of the polyhydronaphthyl moiety in the 6β or equatorial position. Additionally, pharmaceutical compositions of these novel compounds, as the sole

therapeutically active ingredient and in combination with bile acid sequestrants, are disclosed. Other embodiments of this invention are methods of treating disease conditions in which hypercholesterolemia is an etiological factor, and processes for preparing the novel compounds.

## DETAILED DESCRIPTION OF THE INVENTION

The specific HMG-CoA reductase inhibitors of this invention are the compounds represented by the following general structural formulae (I) and (II):

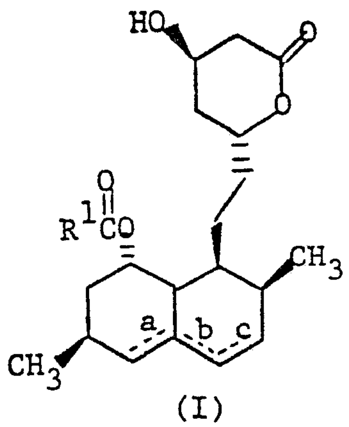

(I)

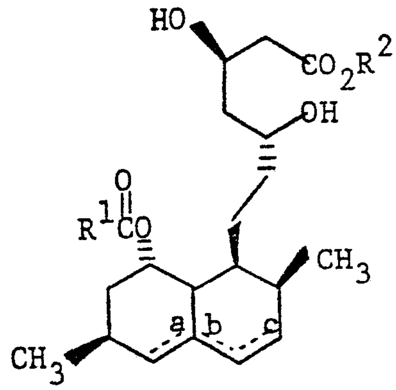

(II)

wherein:
$R^1$ is selected from:
(1) $C_{1-10}$ alkyl;
(2) substituted $C_{1-10}$ alkyl in which one or more substituent(s) is selected from
(a) fluoro,
(b) hydroxy,
(c) $C_{1-10}$ alkoxy,
(d) $C_{1-5}$ alkoxycarbonyl,
(e) $C_{1-5}$ acyloxy,
(f) $C_{3-8}$ cycloalkyl,
(g) phenyl,
(h) substituted phenyl in which the substituents are X and Y,
(i) $C_{1-10}$ alkylS(0)$_n$ in which n is 0 to 2,
(j) $C_{3-8}$ cycloalkylS(0)$_n$,
(k) phenylS(0)$_n$,
(l) substituted phenylS(0)$_n$ in which the substituents are X and Y, and
(m) oxo;
(3) $C_{1-10}$ alkoxy;
(4) $C_{2-10}$ alkenyl;
(5) $C_{3-8}$ cycloalkyl;
(6) substituted $C_{3-8}$ cycloalkyl in which one substituent is selected from
(a) $C_{1-10}$ alkyl
(b) substituted $C_{1-10}$ alkyl in which the substituent is selected from

(i) fluoro,
(ii) hydroxy,
(iii) $C_{1-10}$ alkoxy,
(iv) C $_{1-5}$ alkoxycarbonyl,
(v) $C_{1-5}$ acyloxy
(vi) phenyl,
(vii) substituted phenyl in which the substituents are X and Y
(viii) $C_{1-10}$ alkylS(O)$_n$,
(ix) $C_{3-8}$ cycloalkylS(O)$_n$,
(x) phenylS(O)$_n$,
(xi) substituted phenylS(O)$_n$ in which the substituents are X and Y, and
(xii) oxo,
(c) $C_{1-10}$ alkylS(0)$_n$,

(d) $C_{3-8}$ cycloalkyl$S(0)_n$,
(e) phenyl$S(0)_n$,
(f) substituted phenyl$S(0)_n$ in which the substituents are X and Y,
(g) fluoro,
(h) hydroxy,
(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y;
(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from
(a) piperidinyl,
(b) pyrrolidinyl,
(c) piperazinyl,
(d) morpholinyl, and
(e) thiomorpholinyl; and
(17) $R^5S$ in which $R^5$ is selected from
(a) $C_{1-10}$ alkyl,
(b) phenyl, and
(c) substituted phenyl in which the substituents are X and Y; $R^2$ is selected from:
(1) hydrogen;
(2) $C_{1-5}$ alkyl;
(3) substituted $C_{1-5}$ alkyl in which the substituent is selected from
(a) phenyl,
(b) dimethylamino, and
(c) acetylamino, and
(4) 2,3-dihydroxypropyl; X and Y independently are hydrogen, fluoro, trifluoromethyl, $C_{1-3}$ alkyl, cyano or group selected from:
(1) $R^8O(CH_2)_m$ in which m is 0 to 3 and $R^8$ is hydrogen, $C_{1-3}$alkyl or hydroxy-$C_{2-3}$alkyl;
(2) $R^9\overset{O}{\overset{\|}{C}}O(CH_2)m$ or $R^9O\overset{O}{\overset{\|}{C}}O(CH_2)m$ in which $R^9$ is hydrogen, $C_{1-3}$alkyl, hydroxy-$C_{2-3}$alkyl, phenyl, naphthyl, amino-$C_{1-3}$alkyl, $C_{1-3}$alkylamino-$C_{1-3}$alkyl, di($C_{1-3}$alkyl)amino-$C_{1-3}$alkyl, hydroxy-$C_{2-3}$ alkylamino-$C_{1-3}$alkyl or di(hydroxy-$C_{2-3}$alkyl) amino-$C_{1-3}$alkyl;
(3) $R^{10}O\overset{O}{\overset{\|}{C}}$ $(CH_2)_m$ in which $R^{10}$ is hydrogen, $C_{1-3}$alkyl, hydroxy-$C_{2-3}$alkyl, $C_{1-3}$alkoxy-$C_{1-3}$alkyl, phenyl or naphthyl;
(4) $R^{11}R^{12}N(CH_2)_m$, $R^{11}R^{12}N\overset{O}{\overset{\|}{C}}(CH_2)_m$
or $R^{11}R^{12}N\overset{O}{\overset{\|}{C}}O(CH_2)_m$ in which $R^{11}$ and $R^{12}$ independently are hydrogen, $C_{1-3}$ alkyl, hydroxy-$C_{2-3}$alkyl or together with the nitrogen atom to which they are attached form a heterocyclic group selected from piperidinyl, pyrrolidinyl, piperazinyl, morpholinyl or thiomorpholinyl;
(5) $R^{13}S(0)_n(CH_2)_m$ in which $R^{13}$ is hydrogen, $C_{1-3}$alkyl, amino, $C_{1-3}$alkylamino or di($C_{1-3}$alkyl)amino; and a, b and c each represent single bonds or one of a, b and c represents a double bond or both a and c represent double bonds;
or a pharmaceutically acceptable salt thereof.

Except where specifically defined to the contrary, the terms "alkyl", "alkoxy" and "acyl" include both the straight-chain and branched-chain species of the term.

One embodiment of this invention is the class of compounds of the formulae (I) and (II) wherein: $R^1$ is selected from:
(1) $C_{1-10}$ alkyl;
(2) substituted $C_{1-10}$ alkyl in which one or more substituent(s) is selected from
(a) fluoro,
(b) hydroxy,
(c) $C_{1-10}$ alkoxy,
(d) $C_{1-5}$ alkoxycarbonyl,
(e) $C_{1-5}$ acyloxy,
(f) $C_{3-8}$ cycloalkyl,

(g) phenyl,
(h) substituted phenyl in which the substituents are X and Y, and
(i) oxo;
(3) $C_{3-8}$ cycloalkyl;
(4) substituted $C_{3-8}$ cycloalkyl in which one substituent is selected from
(a) $C_{1-10}$ alkyl,
(b) substituted $C_{1-10}$ alkyl in which the ent is selected from

(i) fluoro,
(ii) hydroxy,
(iii) $C_{1-10}$ alkoxy
(iv) $C_{1-5}$ acyloxy,
(v) $C_{1-5}$ alkoxycarbonyl,
(vi) phenyl,
(vii) substituted phenyl in which the substituents are X and Y, and
(viii) oxo,
(c) fluoro,
(d) hydroxy,
(e) $C_{1-10}$ alkoxy,
(f) $C_{1-5}$ alkoxycarbonyl,
(g) $C_{1-5}$ acyloxy,
(h) phenyl,
(i) substituted phenyl in which the substituents are X and Y;
(5) phenylamino;
(6) substituted phenylamino in which the substituents are X and Y;
(7) phenyl $C_{1-10}$ alkylamino;
(8) substituted phenyl $C_{1-10}$ alkylamino in which the substituents are X and Y.

One subclass of this embodiment is the compounds of the formulae (I) and (II) wherein:
$R^1$ is selected from:
(1) $C_{1-10}$ alkyl;
(2) $C_{3-8}$ cycloalkyl;
(3) phenylamino; and
(4) substituted phenylamino in which the substituents are X and Y.

Exemplifying this subclass are those compounds of the formulae (I) and (II) wherein both a and c represent double bonds, especially the following compounds:
(1) 6(R)-[2-[8(S)-(2,2-dimethylbutyryl-oxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(S)-dimethyl-1,2,6,7,8,8a(R)-hexahydronaphthyl-1(S)]ethyl]-4(R)-hydroxy-3,4,5,6 tetrahydro-2H-pyran-2-one; and the corresponding ring opened dihydroxy acids, and esters thereof.

Also exemplifying this subclass are those compounds of the formulae (I) and (II) wherein each of a, b and c represents single bonds, especially the following compounds:
(1) 6(R)-[2-[8(S)-(2,2-dimethylbutyryloxy)-2(S),6(R)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(R)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one; and the corresponding ring opened dihydroxy acids, and esters.

Another example of this subclass are those compounds of the formulae (I) and (II) wherein one of a, b and c represents single bonds, especially the following compounds:
(1) 6(R)-[2-(8(S)-(2,2-dimethylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one; and the corresponding ring opened dihydroxy acids, and esters thereof.

The compounds of formula (I) wherein $R^1$ does not contain a primary hydroxy group are conveniently prepared from 6(R)-[2-[8(S)-(acyloxy)-2(S)-methyl-6(S)-hydroxymethyl-1,2,6,7,8,8a(R)-hexahydronaphthyl-1(S)]ethyl]-4(R)-hydroxy-3,4,5,6-tetrahydro-2H-pyran-2-one or the tetrahydro or the dihydro analogs thereof via the following synthetic pathway:

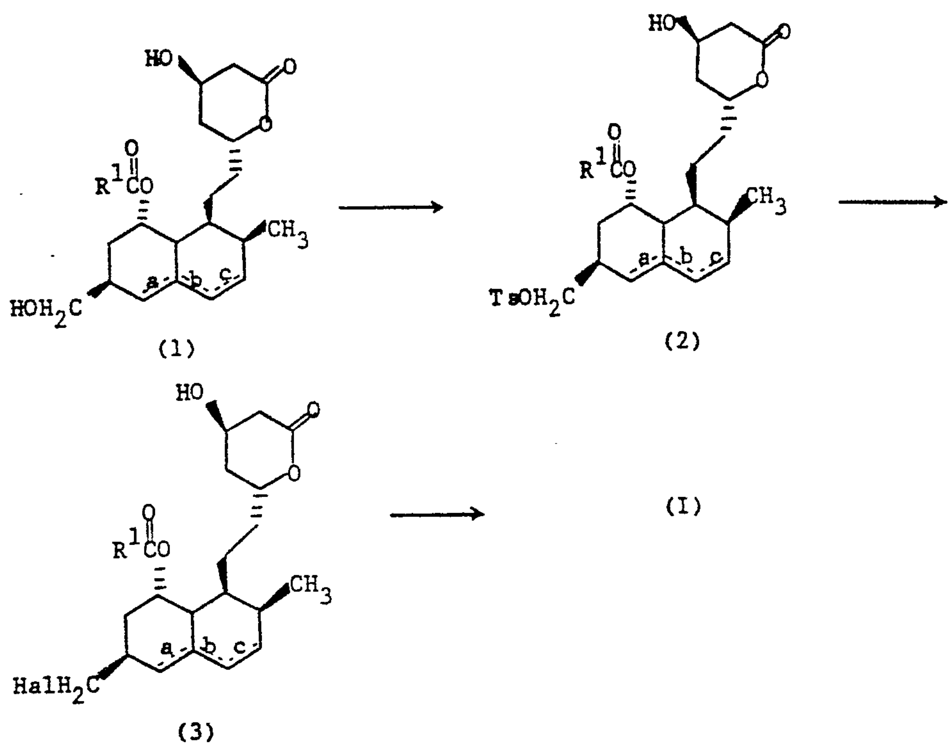

(1) (2) (3) (I)

The compounds of formula (I) including those wherein $R^1$ contains a primary hydroxy group are conveniently prepared from 6(R)-[2-[8(S)-(acyloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one or the tetrahydro or the dihydro analogs thereof by the hydrolysis of the 8-acyloxy group and esterification of the resultant 8-hydroxy group according the the general procedures described in U.S. Patent 4,444,784.

The starting compounds (1) may be prepared according to the general procedure exemplified in co-pending U.S. patent application, Serial No. 048,136 filed May 15, 1987. Compound (1) is reacted with p-toluenesulfonyl chloride in the presence of a base, such as a tertiary amine or an aromatic amine, and an inert solvent, such as a halogenated hydrocarbon to form the tosylate derivative (2). Compound (2) is then reacted with an alkali metal halide, such as sodium iodide, in an inert solvent to afford a halomethyl derivative (3). Compound (3) is reduced with a suitable tin hydride, such as a trialkyltin hydride, in an inert solvent to give the desired compounds of the formula (I).

Where the product formed by the above described synthetic pathways is not the desired form of that compound, then that product may be subjected to one or more further reactions such as hydrolysis, salification, esterification, acylation, ammonolysis or lactonization by conventional methods, as described in more detail hereafter.

Preferred metal salts are salts with alkali metals, such as sodium or potassium, salts with alkaline earth metals, such as calcium, or salts with other metals such as magnesium, aluminum, iron, zinc, copper, nickel or cobalt, of which the alkali metal, alkaline earth metal, magnesium and aluminum salts are preferred, the sodium, calcium and aluminum salts being most preferred.

Preferred amino acids to form amino acid salts are basic amino acids, such as arginine, lysine, histidine, a,β-diaminobutyric acid or ornithine.

Preferred amines to form amine salts include t-octylamine, dibenzylamine, dichlorohexylamine, morpholine, alkyl esters of D-phenylglycine and D-glucosamine. Also preferred is ammonia to form the ammonium salt.

Esters are preferably the alkyl esters, such as the methyl, ethyl, propyl, isopropyl, butyl, isobutyl or pentyl esters, of which the methyl ester is preferred. However, other esters such as phenyl-$C_{1-5}$alkyl, dimethylamino-$C_{1-5}$alkyl, or acetylamino-$C_{1-5}$alkyl may be employed if desired.

Metal salts of the carboxylic acids of formula (II) may be obtained by contacting a hydroxide, carbonate or similar reactive compound of the chosen metal in an aqueous solvent with the carboxylic acid of formula (II). The aqueous solvent employed is preferably water, or it may be a mixture of water with an organic solvent, preferably an alcohol (such as methanol or ethanol), a ketone (such as acetone), an aliphatic hydrocarbon (such as hexane) or an ester (such as ethyl acetate). It is preferred to use a mixture of a hydrophilic organic solvent with water. Such reactions are normally conducted at ambient temperature but they may, if desired, be conducted with heating or cooling.

Amine salts of the carboxylic acids of formula (II) may be obtained by contacting an amine in an aqueous solvent with the carboxylic acid of formula (II). Suitable aqueous solvents include water and mixtures of water

with alcohols (such as methanol or ethanol), ethers (such as tetrahydrofuran), nitriles (such as acetonitrile) or ketones (such as acetone); it is preferred to use aqueous acetone as the solvent for this reaction. The reaction is preferably carried out at a temperature of ambient or below, more preferably a temperature of from 5° to 10° C. The reaction immediately goes to completion. Alternatively, a metal salt of the carboxylic acid of formula (II) (which may have been obtained as described above) can be dissolved in an aqueous solvent, after which a mineral acid salt (for example the hydrochloride) of the desired amine is added, employing the same reaction conditions as when the amine itself is reacted with the carboxylic acid of formula (II) and the desired product is then obtained by a salt exchange reaction.

Amino acid salts of the carboxylic acids of formula (II) may be obtained by contacting an amino acid in aqueous solution with the carboxylic acid of formula (II). Suitable aqueous solvents include water and mixtures of water with alcohols (such as methanol or ethanol) or ethers (such as tetrahydrofuran).

Esters, preferably alkyl esters, of the carboxylic acids of formula (II) may be obtained by contacting the carboxylic acid of formula (II) with an appropriate alcohol, preferably in the presence of an acid catalyst, for example a mineral acid (such as hydrochloric acid or sulphuric acid), a Lewis acid (for example boron trifluoride) or an ion exchange resin. The solvent employed for this reaction is not critical, provided that it does not adversely affect the reaction; suitable solvents include benzene, chloroform, ethers and the like. Alternatively, the desired product may be obtained by contacting the carboxylic acid of formula (II) with a diazoalkane, in which the alkane moiety may be substituted or unsubstituted. This reaction is usually effected by contacting the acid with an ethereal solution of the diazoalkane. As a further alternative, the ester may be obtained by contacting a metal salt of the carboxylic acid of formula (II) with a halide, preferably an alkyl halide, in a suitable solvent; preferred solvents include dimethylformamide, tetrahydrofuran, dimethylsulfoxide and acetone. All of the reactions for producing esters are preferably effected at about ambient temperature, but, if required by the nature of the reaction system, the reactions may be conducted with heating or cooling.

Lactones of the carboxylic acids of formula (I) may be obtained by lactonizing the carboxylic acids of formula (II) under ordinary conditions known to one skilled in the art.

The intrinsic HMG-CoA reductase inhibition activity of the claimed compounds is measured in the in vitro protocol published in J. Med. Chem., 28, p. 347-358 (1985).

For estimation of relative inhibitory potencies, compactin (i.e., mevastatin) was assigned a value of 100 and the $IC_{50}$ value of the test compound was compared with that of compactin determined simultaneously in the published in vitro protocol.

Representative of the intrinsic HMG-CoA reductase inhibitory activities of the claimed compounds is the relative potency for 6(R)-[2-(8(S)-(2,2-dimethylbutylyroxy)-2(S),6(S)-dimethyl-1,2,6,7, 8,8a(R)-hexahydronaphthyl-1(S)]ethyl]-4(R)-hydroxy-3,4,5,6-tetrahydro-2H-pyran-2-one of 225.

The compounds of this invention are useful as antihypercholesterolemic agents for the treatment of arteriosclerosis, hyperlipidemia, familial hypercholesterolemia and like diseases in humans. They may be administered orally or parenterally in the form of a capsule, a tablet, an injectable preparation or the like. It is usually desirable to use the oral route. Doses may be varied, depending on the age, severity, body weight and other conditions of human patients but daily dosage for adults is within a range of from about 10 mg to 2000 mg (preferably 10 to 100 mg) which may be given in two to four divided doses. Higher doses may be favorably employed as required.

The compounds of this invention may also be coadministered with pharmaceutically acceptable nontoxic cationic polymers capable of binding bile acids in a non-reabsorbable form in the gastro-intestinal tract. Examples of such polymers include cholestyramine, colestipol and poly[methyl-(3-trimethylaminopropyl)imino-trimethylene dihalide]. The relative amounts of the compounds of this invention and these polymers is between 1:100 and 1:15,000.

Included within the scope of this invention is the method of treating arteriosclerosis, familial hypercholesterolemia or hyperlipidemia which comprises administering to a subject in need of such treatment a nontoxic, therapeutically-effective amount of the compounds of formulae (I) or (II) or pharmaceutical compositions thereof.

The following examples illustrate the preparation of the compounds of the formulae (I) and (II) and their incorporation into pharmaceutical compositions and as such are not to be considered as limiting the invention set forth in the claims appended hereto.

## EXAMPLE 1

Preparation of 6(R)-[2-[8(S)-(2,2-dimethylbutyryloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one

Step
A. 6(R)-[2-(8(S)-(2,2-dimethylbutyryloxy)-2(S)-methyl-6(S)-(toluenesulfonyloxymethyl)-1,2,6,7,8,8a(R)-hexahydronaphthyl-1(S)]ethyl]-4(R)-hydroxy-3,4,5,6-tetrahydro-2H-pyran-2-one (1a)

To a solution of 6(R)-[2-(8(S)-(2,2-dimethylbutyryloxy)-2(S)-methyl-6(S)-(hydroxymethyl)-1,2,6,7,8,8a(R)-hexahydronaphthyl-1(S)]ethyl]-4(R)-hydroxy-3,4,5, 6-tetrahydro-2H-pyran-2-one (1.0 g, 2.3 mmole) in distilled pyridine (750 µl) under an argon atmosphere was added a solution of p-toluenesulfonyl chloride (0.46 g, 2.4 mmole) in methylene chloride (750 µl). The reaction mixture was stirred at room temperature for about 16 hours and then diluted with methylene chloride (10 ml) and extracted with 1N hydrochloric acid (2 x 10 ml). The organic layer was dried over anhydrous sodium sulfate and then concentrated in vacuo to give an oily residue which contained the desired product.
nmr ($CDCl_3$): δ 0.83 (t,J = 7.5Hz, 3H); 0.90 (d,J = 7.5 Hz, 3H); 1.13 (s,6H); 2.63 (m, 1H); 2.75 (dd,J = 17.4, 4.8 Hz, 1H); 3.93 (d, J = 6.0 Hz, 2H); 4.38 (m, 1H); 4.62 (m, 1H); 5.39 (m, 1H); 5.52 (m, 1H); 5.81 (dd, J = 9.7, 6.0 Hz, 1H); 5.92 (d, J = 9.8 Hz, 1H); 7.37 (d, J = 8.0 Hz, 2H); 7.79 (d, J = 8.0 Hz, 2H).

Step
B. 6(R)-[2-(8(S)-(2,2-dimethylbutyryloxy)-2(S)-methyl-6(S)-(iodomethyl)-1,2,6,7,8,8a(R)-hexahydronaphthyl-1(S)]ethyl]-4(R)-hydroxy-3,4,5,6-tetrahydro-2H-pyran-2-one (1b)

To a solution of the crude compound 1a in acetone under an argon atmosphere was added sodium iodide (0.79 g, 5.3 mmole) and the resultant mixture refluxed for 4 hours. The solvent was removed in vacuo and the residue partitioned between ethyl acetate and water. The organic phase was washed with aqueous sodium bisulfite (2x), brine and dried over anhydrous sodium sulfate and concentrated in vacuo to yield an oil. The crude oil was purified by flash chromatography over silica gel eluted with a gradient of 10 to 50 percent ethyl acetate in hexane to afford an gummy solid which crystallized upon trituration with hexane. nmr ($CDCl_3$): δ 0.83 (t,J = 7.5 Hz, 3H); 0.90 (d,J = 7.1 Hz, 3H); 1.13(s,6H); 2.63 (m, 1H); 2.75 (dd,J = 17.4, 4.8 Hz, 1H); 3.19 (d, J = 4.8 Hz, 2H); 4.38 (m, 1H); 4.62 (m, 1H); 5.35 (m, 1H); 5.40 (m, 1H); 5.85 (dd, J = 9.7, 6.0 Hz, 1H); 6.01 (d, J = 9.8 Hz, 1H).
Elemental Anal. Calc'd for $C_{25}H_{37}IO_5$:
C, 55.15; H, 6.85.
Found: C, 55.45; H, 7.00.

Step
C. 6(R)-[2-[8(S)-(2,2-dimethylbutyryloxy)-2(S),6(S)-dimethyl-1,2,6,7,8,8a(R)-hexahydronaphthyl-1(S)]-ethyl]-4(R)-hydroxy-3,4,5,6-tetrahydro-2H-pyran-2-one

To a solution of the compound 1b (212 mg, 0.39 mmole) in benzene under an argon atmosphere was added tributyltin hydride (110 µl, 0.41 mmole). The reaction mixture was stirred at room temperature for about 16 hours. To the reaction mixture was then added dropwise a solution of iodine in benzene until the purple color persisted. The reaction mixture was then washed with aqueous sodium bisulfite and brine and dried over anhydrous sodium sulfate. Removal of the solvent in vacuo afforded an oil which was flash chromatographed over silica gel eluted with a gradient of 10 to 50 percent ethyl acetate in hexane to yield an oil which crystallized upon trituration with ligroine. mp 90-96° C, nmr ($CDCl_3$): δ 0.83 (t,J = 7.5 Hz, 3H); 0.90 (d,J = 7.1 Hz, 3H); 1.02 (d, J = 7.1 Hz, 3H); 1.13 (s,6H); 2.63 (m, 1H); 2.75 (dd,J = 17.4, 4.8 Hz, 1H); 4.38 (m, 1H); 4.62 (m, 1H); 5.29 (m, 1H); 5.41 (m, 1H); 5.76 (dd, J = 9.7, 6.0 Hz, 1H); 5.98 (d, J = 9.8 Hz, 1H);
Elemental Anal. Calc'd for $C_{25}H_{38}O_5$:
C, 71.74; H, 9.15.
Found: C, 71.85; H, 9.17.

## Examples 2 to 6

Utilizing the general procedures of Example 1 but substituting the appropriate starting materials and reactants, the following compounds are prepared:

(2) 6(R)-[2-[8(S)-(2(S)-methylbutyryloxy-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one;

(3) 6(R)-[2-[8(S)-(2,2-dimethylbutyryloxy)-2(S),6(R)-dimethyl-1,2,3,4,4a(S),5,6, 7,8,8a(S)-decahydronaphthyl-1(S)]ethyl]-4(R)-hydroxy-3,4,5,6-tetrahydro-2H-pyran-2-one;

(4) 6(R)-[2-[8(S)-(2(S)-methylbutyryloxy)-2(S),6(R)-dimethyl-1,2,3,4,4a(S),5,6,7,8,8a-(S)-decahydronaphthyl-1(S)]ethyl]-4(R)-hydroxy-3,4,5,6-tetrahydro-2H-pyran-2-one;

(5) 6(R)-[2-(8(S)-(2,2-dimethylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one; and

(6) 6(R)-[2-[8(S)-(2-methylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one.

EXAMPLE 7

Preparation of Ammonium Salts of Compounds II

The lactone (1.0 mmol) from Example 1 Step C is dissolved with stirring in 0.1N NaOH (1.1 mmol) at ambient temperature. The resulting solution is cooled and acidified by the dropwise addition of 1N HCl. The resulting mixture is extracted with diethyl ether and the extract washed with brine and dried ($MgSO_4$). The $MgSO_4$ is removed by filtration and the filtrate saturated with ammonia (gas) to give a gum which solidified to provide the ammonium salt.

EXAMPLE 8

Preparation of Alkali and Alkaline Earth Salts of Compounds II

To a solution of 42 mg of lactone from Example 1 Step C in 2 ml of ethanol is added 1 ml of aqueous NaOH (1 equivalent). After one hour at room temperature, the mixture is taken to dryness in vacuo to yield the desired sodium salt.

In like manner, the potassium salt is prepared using one equivalent of potassium hydroxide, and the calcium salt, using one equivalent of CaO.

EXAMPLE 9

Preparation of Ethylenediamine Salts of Compounds II

To a solution of 0.50 g of the ammonium salt from Example 7 in 10 ml of methanol is added 75 ml of ethylenediamine. The methanol is stripped off under vacuum to obtain the desired ethylenediamine salt.

EXAMPLE 10

Preparation of Tris(hydroxymethyl)aminomethane Salts of Compounds II

To a solution of 202 mg of the ammonium salt from Example 7 in 5 ml of methanol is added a solution of 60.5 mg of tris(hydroxymethyl) aminomethane in 5 ml of methanol. The solvent is removed in vacuo to afford the desired tris(hydroxymethyl)aminomethane salt.

EXAMPLE 11

Preparation of L-Lysine Salts of Compounds II

A solution of 0.001 mole of L-lysine and 0.0011 mole of the ammonium salt from Example 7 in 15 ml of 85% ethanol is concentrated to dryness in vacuo to give the desired L-lysine salt.

Similarly prepared are the L-arginine, L-ornithine, and N methylglucamine salts.

EXAMPLE 12

Preparation of Tetramethylammonium Salts of Compounds II

A mixture of 68 mg of ammonium salt from Example 7 in 2 ml of methylene chloride and 0.08 ml of 24% tetramethylammonium hydroxide in methanol is diluted with ether to yield the desired tetramethylammoniun salt.

EXAMPLE 13

Preparation of Methyl Esters of Compounds II

To a solution of 400 mg of lactone from Example 1 Step C in 100 ml of absolute methanol is added 10 ml 0.1 M sodium methoxide in absolute methanol. This solution is allowed to stand at room temperature for one hour, then is diluted with water and extracted twice with ethyl acetate. The organic phase is separated, dried ($Na_2SO_4$), filtered and evaporated in vacuo to yield the desired methyl ester.

In like manner, by the use of equivalent amounts of propanol, butanol, isobutanol, t-butanol, amylalcohol, isoamylalcohol, 2-dimethylaminoethanol, benzylalcohol, phenethanol, 2-acetamidoethanol and the like, the corresponding esters are obtained.

EXAMPLE 14

Preparation of Free Dihydroxy Acids

The sodium salt of the compound II from Example 8 is dissolved in 2 ml of ethanol-water (1:1; v:v) and added to 10 ml of 1N hydrochloric acid from which the dihydroxy acid is extracted with ethyl acetate. The organic extract is washed once with water, dried ($Na_2SO_4$), and evaporated in vacuo with a bath temperature not exceeding 30°C. The dihydroxy acid derivative derived slowly reverts to the corresponding, parent lactone on standing.

EXAMPLE 15

As a specific embodiment of a composition of this invention, 20 mg of lactone from Example 1 Step C is formulated with sufficient finely divided lactose to provide a total amount of 580 to 590 mg to fill a size 0, hard-gelatin capsule.

**Claims**

1. A compound represented by the following general structural formulae (I) and (II):

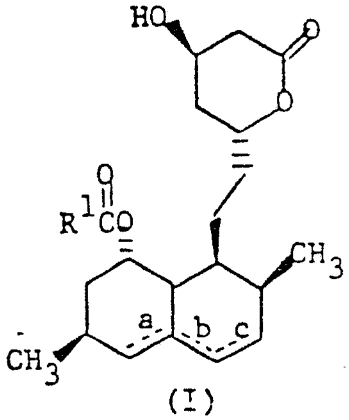

(I)

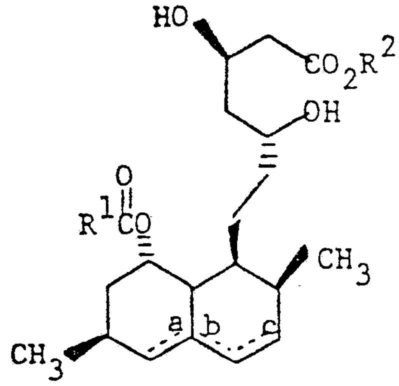

(II)

wherein:
$R^1$ is selected from:
(1) $C_{1-10}$ alkyl;
(2) substituted $C_{1-10}$ alkyl in which one or more substituent(s) is selected from

(a) fluoro,
(b) hydroxy,
(c) $C_{1-10}$ alkoxy,
(d) $C_{1-5}$ alkoxycarbonyl
(e) $C_{1-5}$ acyloxy,
(f) $C_{3-8}$ cycloalkyl,
(g) phenyl,
(h) substituted phenyl in which the substituents are X and Y,
(i) $C_{1-10}$ alkylS(O)$_n$ in which n is 0 to 2,
(j) $C_{3-8}$ cycloalkylS(O)$_n$,
(k) phenylS(O)$_n$,
(l) substituted phenylS(O)$_n$ in which the substituents are X and Y, and
(m) oxo;

(3) $C_{1-10}$ alkoxy;
(4) $C_{2-10}$ alkenyl;
(5) $C_{3-8}$ cycloalkyl;
(6) substituted $C_{3-8}$ cycloalkyl in which one substituent is selected from

(a) $C_{1-10}$ alkyl
(b) substituted $C_{1-10}$ alkyl in which the substituent is selected from

(i) fluoro,
(ii) hydroxy,
(iii) $C_{1-10}$ alkoxy,
(iv) $C_{1-5}$ alkoxycarbonyl,
(v) $C_{1-5}$ acyloxy
(vi) phenyl,
(vii) substituted phenyl in which the substituents are X and Y
(viii) $C_{1-10}$ alkylS(O)$_n$,
(ix) $C_{3-8}$ cycloalkylS(O)$_n$,
(x) phenylS(O)$_n$,
(xi) substituted phenylS(O)$_n$ in which the substituents are X and Y, and
(xii) oxo,

(c) $C_{1-10}$ alkylS(O)$_n$,
(d) $C_{3-8}$ cycloalkylS(O)$_n$,
(e) phenylS(O)$_n$,
(f) substituted phenylS(O)$_n$ in which the substituents are X and Y,
(g) fluoro,
(h) hydroxy,

(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y;

(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from

(a) piperidinyl,
(b) pyrrolidinyl,
(c) piperazinyl,
(d) morpholinyl, and
(e) thiomorpholinyl; and
(17) $R^5S$ in which $R^5$ is selected from

(a) $C_{1-10}$ alkyl,
(b) phenyl, and
(c) substituted phenyl in which the substituents are X and Y;

$R^2$ is selected from:

(1) hydrogen;
(2) $C_{1-5}$ alkyl;
(3) substituted $C_{1-5}$ alkyl in which the substituent is selected from

(a) phenyl,
(b) dimethylamino, and
(c) acetylamino, and

(4) 2,3-dihydroxypropyl;
X and Y independently are hydrogen, fluoro, trifluoromethyl, $C_{1-3}$ alkyl, cyano or group selected from:
(1) $R^8O(CH_2)_m$ in which m is 0 to 3 and $R^8$ is hydrogen, $C_{1-3}$alkyl or hydroxy-$C_{2-3}$alkyl;
(2) $R^9\overset{O}{\overset{\|}{C}}O(CH_2)_m$ or $R^9O\overset{O}{\overset{\|}{C}}O(CH_2)_m$ in which $R^9$ is hydrogen, $C_{1-3}$alkyl, hydroxy-$C_{2-3}$alkyl, phenyl, naphthyl, amino-$C_{1-3}$alkyl, $C_{1-3}$alkylamino-$C_{1-3}$alkyl, di($C_{1-3}$alkyl)amino-$C_{1-3}$alkyl, hydroxy-$C_{2-3}$ alkylamino-$C_{1-3}$alkyl or di(hydroxy-$C_{2-3}$alkyl) amino-$C_{1-3}$alkyl;
(3) $R^{10}O\overset{O}{\overset{\|}{C}}(CH_2)_m$ in which $R^{10}$ is hydrogen, $C_{1-3}$alkyl, hydroxy-$C_{2-3}$ alkyl, $C_{1-3}$alkoxy-$C_{1-3}$alkyl, phenyl or naphthyl;
(4) $R^{11}R^{12}N(CH_2)_m$, $R^{11}R^{12}N\overset{O}{\overset{\|}{C}}(CH_2)_m$

or $R^{11}R^{12}N\ \ O(CH_2)_m$ in which $R^{11}$ and $R^{12}$ independently are hydrogen, $C_{1-3}$ alkyl, hydroxy-$C_{2-3}$alkyl or together with the nitrogen atom to which they are attached form a heterocyclic group selected from piperidinyl, pyrrolidinyl, piperazinyl, morpholinyl or thiomorpholinyl;
(5) $R^{13}S(O)_n(CH_2)_m$ in which $R^{13}$ is hydrogen, $C_{1-3}$alkyl, amino, $C_{1-3}$alkylamino or di($C_{1-3}$alkyl)amino; and
$\underline{a}$, $\underline{b}$ and $\underline{c}$ each represent single bonds or one of $\underline{a}$, $\underline{b}$ and $\underline{c}$ represents a double bond or both $\underline{a}$ and $\underline{c}$represent double bonds;
or a pharmaceutically acceptable salt thereof.

2. A compound of Claim 1 wherein:
$R^1$ is selected from:
(1) $C_{1-10}$ alkyl;
(2) substituted $C_{1-10}$ alkyl in which one or more substituent(s) is selected from

(a) fluoro,
(b) hydroxy,

(c) $C_{1-10}$ alkoxy,
(d) $C_{1-5}$ alkoxycarbonyl,
(e) $C_{1-5}$ acyloxy,
(f) $C_{3-8}$ cycloalkyl,
(g) phenyl,
(h) substituted phenyl in which the substituents are X and Y, and
(i) oxo;
(3) $C_{3-8}$ cycloalkyl;
(4) substituted $C_{3-8}$ cycloalkyl in which one substituent is selected from

(a) $C_{1-10}$ alkyl,
(b) substituted $C_{1-10}$ alkyl in which the substituent is selected from

(i) fluoro,
(ii) hydroxy,
(iii) $C_{1-10}$ alkoxy
(iv) $C_{1-5}$ acyloxy,
(v) $C_{1-5}$ alkoxycarbonyl,
(vi) phenyl,
(vii) substituted phenyl in which the substituents are X and Y, and
(viii) oxo,

(c) fluoro,
(d) hydroxy,
(e) $C_{1-10}$ alkoxy,
(f) $C_{1-5}$ alkoxycarbonyl,
(g) $C_{1-5}$ acyloxy,
(h) phenyl,
(i) substituted phenyl in which the substituents are X and Y;
(5) phenylamino;
(6) substituted phenylamino in which the substituents are X and Y;
(7) phenyl $C_{1-10}$ alkylamino;
(8) substituted phenyl $C_{1-10}$ alkylamino in which the substituents are X and Y.

3. A compound of Claim 2 wherein:
$R^1$ is selected from:
(1) $C_{1-10}$ alkyl; and
(2) $C_{3-8}$ cycloalkyl.

4. A compound of Claim 3 wherein a and c represent double bonds.

5. A compound of Claim 4 which is selected from:

(1) 6(R)-[2-[8(S)-(2,2-dimethylbutyryloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one; and
the corresponding ring opened dihydroxy acids, and esters thereof.

6. A compound of Claim 3 wherein a, b and c represent single bonds or one of a, b or c represents a double bond.

7. A compound of Claim 6 which is selected from:

(1) 6(R)-[2-[8(S)-(2,2-dimethylbutyryloxy)-2(S),6(R)-dimethyl-1,2,3,4,4a(S),5,6, 7,8,8a(S)-decahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(R)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one;
(3) 6(R)-[2-(8(S)-(2,2-dimethylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one;
(4) 6(R)-[2-[8(S)-(2(S)-methylbutyryloxy)-2(S),6(S)-dimethyl-1,2,3,4,6,7,8,8a(R)-octahydro naphthyl-1(S)]ethyl]-4(R)-hydroxy-3,4,5,6-tetrahydro-2H-pyran-2-one; and
the corresponding ring opened dihydroxy acids, and esters thereof.

8. A hypocholesterolemic, hypolipidemic pharmaceutical composition comprising a pharmaceutically acceptable carrier and a nontoxic effective amount of a compound as defined in Claim 1.

9. A composition of Claim 10 in which the compound is selected from:

(1) 6(R)-[2-[8(S)-(2,2-dimethylbutyryloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one;
(3) 6(R)-[2-[8(S)-(2,2-dimethylbutyryloxy)-2(S),6(R)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one;
(4) 6(R)-[2-[8(S)-(2(S)-methylbutyryloxy)-2(S),6(R)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one;
(5) 6(R)-[2-(8(S)-(2,2-dimethylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one;
(6) 6(R)-[2-[8(S)-(2(S)-methylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one; and
the corresponding ring opened dihydroxy acids, and esters thereof.

10. A process for the preparation of the compound represented by the general structural formula (I) according to Claim 1 which comprises:

(A) treating a compound represented by the following general structural formula (3):

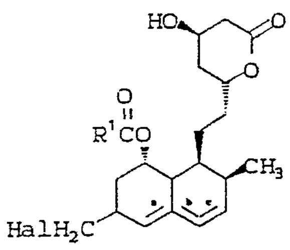

(3)

wherein Hal is iodo, with a trialkyl tin hydride in an inert solvent; and

(B) optionally forming the compound represented by the general structural formula (II) or a pharmaceutically acceptable salt thereof by treating the compound represented by the general structural formula (I) with:
(1) an alkali metal hydroxide in an aqueous solvent to prepare the alkali metal salt; or
(2) an alkali metal hydroxide in an aqueous solvent to prepare the alkali metal salt and acidifying the salt to prepare the free dihydroxy acid; or
(3) an alkali metal hydroxide in an aqueous solvent to prepare the alkali metal salt and esterifying the salt with a $C_{1-5}$ aklanol to prepare the $C_{1-5}$ alkyl ester.

**Claims for the following Contracting States : ES; GR**

1. A process for the preparation of the compound represented by the following general structural formula (I):

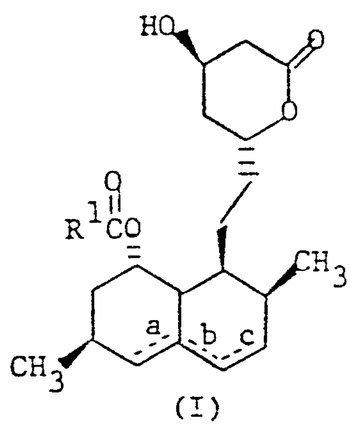

(I)

wherein:
$R^1$ is selected from:
(1) $C_{1-10}$ alkyl;
(2) substituted $C_{1-10}$ alkyl in which one or more substituent(s) is selected from

(a) fluoro,

(b) hydroxy,
(c) $C_{1-10}$ alkoxy,
(d) $C_{1-5}$ alkoxycarbonyl,
(e) $C_{1-5}$ acyloxy,
(f) $C_{3-8}$ cycloalkyl,
(g) phenyl,
(h) substituted phenyl in which the substituents are X and Y,
(i) $C_{1-10}$ alkylS(O)$_n$ in which n is 0 to 2,
(j) $C_{3-8}$ cycloalkylS(O)$_n$,
(k) phenylS(O)$_n$,
(l) substituted phenylS(O)$_n$ in which the substituents are X and Y, and
(m) oxo;

(3) $C_{1-10}$ alkoxy;
(4) $C_{2-10}$ alkenyl;
(5) $C_{3-8}$ cycloalkyl;
(6) substituted $C_{3-8}$ cycloalkyl in which one substituent is selected from

(a) $C_{1-10}$ alkyl
(b) substituted $C_{1-10}$ alkyl in which the substituent is selected from

(i) fluoro,
(ii) hydroxy,
(iii) $C_{1-10}$ alkcxy,
(iv) $C_{1-5}$ alkoxycarbonyl,
(v) $C_{1-5}$ acyloxy
(vi) phenyl,
(vii) substituted phenyl in which the substituents are X and Y
(viii) $C_{1-10}$ alkylS(O)$_n$,
(ix) $C_{3-8}$ cycloalkylS(O)$_n$,
(x) phenylS(O)$_n$,
(xi) substituted phenylS(O)$_n$ in which the substituents are X and Y, and
(xii) oxo,

(c) $C_{1-10}$ alkylS(O)$_n$,
(d) $C_{3-8}$ cycloalkylS(O)$_n$,
(e) phenylS(O)$_n$,
(f) substituted phenylS(O)$_n$ in which the substituents are X and Y,
(g) fluoro,
(h) hydroxy,
(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y;

(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from

(a) piperidinyl,
(b) pyrrolidinyl,
(c) piperazinyl,
(d) morpholinyl, and
(e) thiomorpholinyl; and

(17) $R^5$S in which $R^5$ is selected from

(a) $C_{1-10}$ alkyl,
(b) phenyl, and
(c) substituted phenyl in which the substituents are X and Y;
$R^2$ is selected from:
(1) hydrogen;
(2) $C_{1-5}$ alkyl;
X and Y independently are hydrogen, fluoro, trifluoromethyl, $C_{1-3}$ alkyl, cyano or group selected from:
(1) $R^8O(CH_2)_m$ in which m is 0 to 3 and $R^8$ is hydrogen, $C_{1-3}$alkyl or hydroxy-$C_{2-3}$alkyl;
(2) $R^9\overset{O}{\overset{||}{C}}O(CH_2)_m$ or $R^9O\overset{O}{\overset{||}{C}}O(CH_2)m$ in which $R^9$ is hydrogen, $C_{1-3}$alkyl, hydroxy-$C_{2-3}$alkyl, phenyl, naphthyl, amino-$C_{1-3}$alkyl, $C_{1-3}$alkylamino-$C_{1-3}$alkyl, di($C_{1-3}$alkyl)amino-$C_{1-3}$alkyl, hydroxy-$C_{2-3}$ alkylamino-$C_{1-3}$alkyl or di(hydroxy-$C_{2-3}$alkyl) amino-$C_{1-3}$alkyl;
(3) $R^{10}O\overset{O}{\overset{||}{C}}(CH_2)_m$ in which $R^{10}$ is hydrogen, $C_{1-3}$alkyl, hydroxy-$C_{2-3}$ alkyl, $C_{1-3}$alkoxy-$C_{1-3}$alkyl, phenyl or naphthyl;
(4) $R^{11}R^{12}N(CH_2)_m$, $R^{11}R^{12}N\overset{O}{\overset{||}{C}}(CH_2)_m$ or $R^{11}R^{12}N\overset{O}{\overset{||}{C}}O(CH_2)_m$ in which $R^{11}$ and $R^{12}$ independently are hydrogen, $C_{1-3}$ alkyl, hydroxy-$C_{2-3}$alkyl or together with the nitrogen atom to which they are attached form a heterocyclic group selected from piperidinyl, pyrrolidinyl, piperazinyl, morpholinyl or thiomorpholinyl;
(5) $R^{13}S(O)_n(CH_2)_m$ in which $R^{13}$ is hydrogen, $C_{1-3}$alkyl, amino, $C_{1-3}$alkylamino or di($C_{1-3}$alkyl)amino; and <u>a</u>, <u>b</u> and <u>c</u> each represent single bonds or one of <u>a</u>, <u>b</u> and <u>c</u> represents a double bond or both <u>a</u> and <u>c</u> represent double bonds;
which comprises:
(A) treating a compound represented by the following general structural formula (3):

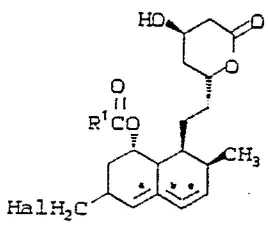

(3)

wherein Hal is iodo, with a trialkyl tin hydride in an inert solvent; and
(B) optionally forming the compound represented by the general structural formula (II) or a pharmaceutically acceptable salt thereof by treating the compound represented by the general structural formula (I) with:
(1) an alkali metal hydroxide in an aqueous solvent to prepare the alkali metal salt; or
(2) an alkali metal hydroxide in an aqueous solvent to prepare the alkali metal salt and acidifying the salt to prepare the free dihydroxy acid; or
(3) an alkali metal hydroxide in an aqueous solvent to prepare the alkali metal salt and esterifying the salt with a $C_{1-5}$ aklanol to prepare the $C_{1-5}$ alkyl ester.
2. A process of Claim 1 wherein: $R^1$ is selected from:
(1) $C_{1-10}$ alkyl;
(2) substituted $C_{1-10}$ alkyl in which one or more substituent(s) is selected from

(a) fluoro,
(b) hydroxy,
(c) $C_{1-10}$ alkoxy,
(d) $C_{1-5}$ alkoxycarbonyl,
(e) $C_{1-5}$ acyloxy,
(f) $C_{3-8}$ cycloalkyl,
(g) phenyl,
(h) substituted phenyl in which the substituents are X and Y, and
(i) oxo;
(3) $C_{3-8}$ cycloalkyl;
(4) substituted $C_{3-8}$ cycloalkyl in which one substituent is selected from

(a) $C_{1-10}$ alkyl,

(b) substituted $C_{1-10}$ alkyl in which the substituent is selected from

(i) fluoro,
(ii) hydroxy,
(iii) $C_{1-10}$ alkoxy
(iv) $C_{1-5}$ acyloxy,
(v) $C_{1-5}$ alkoxycarbonyl,
(vi) phenyl,
(vii) substituted phenyl in which the substituents are X and Y, and
(viii) oxo,

(c) fluoro
(d) hydroxy,
(e) $C_{1-10}$ alkoxy,
(f) $C_{1-5}$ alkoxycarbonyl,
(g) $C_{1-5}$ acyloxy,
(h) phenyl,
(i) substituted phenyl in which the substituents are X and Y;
(5) phenylamino;
(6) substituted phenylamino in which the substituents are X and Y;
(7) phenyl $C_{1-10}$ alkylamino;
(8) substituted phenyl $C_{1-10}$ alkylamino in which the substituents are X and Y.

3. A process of Claim 2 wherein:
$R^1$ is selected from:
(1) $C_{1-10}$ alkyl; and
(2) $C_{3-8}$ cycloalkyl.

4. A process of Claim 3 wherein a and c represent double bonds.

5. A prosecc of Claim 4 which is selected from:
(1) 6(R)-[2-[8(S)-(2,2-dimethylbutyryloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(S)-dimethyl-1,2,6,7,8,8a(R)-hexahydronaphthyl-1(S)]ethyl]-4(R)-hydroxy-3,4,5,6-tetrahydro-2H-pyran-2-one; and
the corresponding ring opened dihydroxy acids, and esters thereof.

6. A process of Claim 3 wherein a, b and c represent single bonds or one of a, b or c represents a double bond.

7. A process of Claim 6 which is selected from:
(1) 6(R)-[2-[8(S)-(2,2-dimethylbutyryl-oxy)-2(S),6(R)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one;
(2) 6(R)-[2-[8(S)-(2(S)-methylbutyryloxy)-2(S),6(R)-dimethyl-1,2,3,4,4a(S),5,6,7,8,8a-(S)-decahydronaphthyl-1(S)]ethyl]-4(R)-hydroxy-3,4,5,6-tetrahydro-2H-pyran-2-one;
(3) 6(R)-[2-(8(S)-(2,2-dimethylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one;
(4) 6(R)-[2-[8(S)-(2(S)-methylbutyryloxy)-2(S),6(S)-dimethyl-1,2,3,4,6,7,8,8a(R)-octahydronaphthyl-1(S)]ethyl]-4(R)-hydroxy-3,4,5,6-tetrahydro-2H-pyran-2-one; and
the corresponding ring opened dihydroxy acids, and esters thereof.